# EUROPÄISCHE PATENTSCHRIFT

(11) **EP 2 582 336 B1**
(45) Veröffentlichungstag und Bekanntmachung des Hinweises auf die Patenterteilung: **11.02.2015**
(21) Anmeldenummer: 11833606.4
(22) Anmeldetag: 06.12.2011
(51) Int. Cl.: A61F 9/007, A61B 17/32

(54) **HOHLNADEL FÜR EIN AUGENCHIRURGISCHES INSTRUMENT**
HOLLOW NEEDLE FOR AN OPHTHALMOLOGICAL INSTRUMENT
AIGUILLE CREUSE POUR UN INSTRUMENT DE LA CHIRURGIE DE L'OEIL

(30) Priorität: 10.02.2011 DE 102011010943
(43) Veröffentlichungstag der Anmeldung: 24.04.2013
(73) Patentinhaber: Geuder AG, 69126 Heidelberg (DE)
(72) Erfinder: FRAUENFELD, Dieter, 69121 Heidelberg (DE); DRAHEIM, René, 69207 Sandhausen (DE); ENGEL, Stefan, 69124 Heidelberg (DE)
(74) Vertreter: Ullrich & Naumann
(86) Internationale Anmeldenummer: PCT/DE2011/050053
(87) Internationale Veröffentlichungsnummer: WO 2012/107009

(56) Entgegenhaltungen:
- WO-A1-99/65405
- DE-A1- 19 942 693
- DE-C1- 19 646 881
- US-A- 6 159 175
- US-A1- 2010 087 846

## Beschreibung

Die Erfindung betrifft eine Hohlnadel für ein augenchirurgisches Instrument zur In-vivo-Zertrümmerung organischer Linsen mittels Ultraschall, mit einem Anschlussbereich zum Ankoppeln an das Instrument und einem am freien Ende ausgebildeten Arbeitsbereich mit einer Wirkfläche zum Abstrahlen von Ultraschallwellen, wobei sich durch die Hohlnadel hindurch ein im Arbeitsbereich offener Absaugkanal zum Absaugen von Linsentrümmern erstreckt, dessen Öffnung durch die Wirkfläche gebildet bzw. begrenzt ist.
Eine gattungsbildende Hohlnadel gemäß Oberbegriff des Anspruchs 1 ist aus der DE 196 46 881 C1 bekannt. Im Konkreten handelt es sich dabei um eine Hohlnadel für ein augenchirurgisches Instrument zur In-vivo-Zertrümmerung von Linsen durch Hochfrequenzbetätigung der Hohlnadel, wobei die Hohlnadel gleichzeitig zum Absaugen von Linsen durch einen inneren Absaugkanal dient. Die Hohlnadel umfasst ein ringförmig ausgebildetes Stirnende, das die Öffnung eines Absaugkanals bildet.

Ultraschallbetätigte Hohlnadeln der gattungsbildenden Art werden bei Kataraktoperationen in der Augenchirurgie verwendet. Das freie Ende der Hohlnadel wird in eine hochfrequente Axialbewegung versetzt und unmittelbar an den Katarakt herangeführt. Vom ringförmigen Stirnende werden Ultraschallwellen zur Emulsifikation des Gewebes abgestrahlt. Abgetrennte Linsenteile bzw. Linsentrümmer werden durch die Hohlnadel hindurch gemeinsam mit einer dem Auge zugeführten Spülflüssigkeit abgeführt.

Zur Verstärkung des emittierten Ultraschallfeldes ist es bereits bekannt, das stirnseitige bzw. freie Ende der Hohlnadel, d.h. die dortige Wirkfläche, gezahnt auszugestalten, um nämlich die Wirkfläche durch die Zahnung zu vergrößern. Die zum Abstrahlen von Ultraschallwellen dienende Wirkfläche wird dadurch vergrößert, so dass die Effizienz des Instruments bzw. der Hohlnadel verbessert ist.

Die aus der DE 196 46 881 C1 bekannte Hohlnadel ist in der Praxis jedoch problematisch, da nicht selten größere Linsentrümmer abgesaugt werden, die im Bereich der Absaugöffnung zu einer zumindest momentanen Verstopfung führen. Dadurch kann im Absaugkanal ein höherer Unterdruck entstehen. Wird der Linsentrümmer schließlich abgesaugt, so wird die Öffnung des Absaugkanals schlagartig wieder frei. Dabei entsteht im Bereich des freien Endes der Hohlnadel ein zu hoher Unterdruck, wodurch erhebliche Beschädigungen des umliegenden Gewebes auftreten können. Ein weiteres Problem ist, dass sich die Dauer des Eingriffs in nicht unerheblichem Maße erhöht, wenn die Absaugöffnung immer wieder verstopft. Da dieser Eingriff am Auge äußerst unangenehm ist, sollte er von möglichst kurzer Dauer sein.

Der vorliegenden Erfindung liegt daher die Aufgabe zugrunde, eine Hohlnadel für ein augenchirurgisches Instrument der gattungsbildenden Art derart auszugestalten und weiterzubilden, dass mit konstruktiv einfachen Mitteln die Zertrümmerung organischer Linsen unter Vermeidung von Verstopfungen des Absaugkanals in geringer Zeit ermöglicht wird.

Die voranstehende Aufgabe ist erfindungsgemäß durch eine Hohlnadel für ein augenchirurgisches Instrument zur In-vivo-Zertrümmerung organischer Linsen mittels Ultraschall mit den Merkmalen des Patentanspruchs 1 gelöst.

Es ist erkannt worden, dass sich die Effizienz der Hohlnadel durch eine einfache konstruktive Maßnahme spürbar erhöhen lässt, nämlich durch ein Wirkelement, das sich zumindest geringfügig in den Absaugkanal hinein erstreckt. Hierbei sei angemerkt, dass "in den Absaugkanal hinein" im weitesten Sinne zu verstehen ist. Das Wirkelement kann direkt an der Öffnung der Hohlnadel oder tiefer im Absaugkanal vorgesehen sein. Mit anderen Worten ist im Arbeitsbereich ein weiteres Wirkelement vorgesehen, durch das zum einen die ultraschallemittierende Fläche maximiert wird und zum anderen Linsenteile nochmals mechanisch zertrümmert werden. Beide Faktoren wirken sich positiv auf die Effizienz der Hohlnadel aus. Auf den ersten Blick erscheint die Maßnahme ein weiteres Element in den bzw. vor dem Absaugkanal anzubringen kontraproduktiv, da der Durchgang somit verkleinert wird. Jedoch lässt sich die Wirkung der Hohlnadel durch diese Konstruktive Maßnahme um ein Vielfaches verbessern. Eine Verstopfung des Absaugkanals durch unzureichend zerkleinerte Linsentrümmer wird dadurch nahezu vermieden.

An dieser Stelle sei darauf hingewiesen, dass im Arbeitsbereich mehrere der erfindungsgemäßen Wirkelemente hintereinander oder eine Kombination unterschiedlicher Wirkelemente vorgesehen sein können. Der Einfachheit halber sei fortan lediglich von "dem Wirkelement" die Rede.

Das Wirkelement ist gegenüber der Hohlnadel beweglich. Durch die Bewegung der Hohlnadel kann das Wirkelement beispielsweise in Rotation oder eine andere Art von Bewegung versetzt werden. Dadurch wird die mechanische Zertrümmerung des Linsenkörpers verstärkt. Des Weiteren ist denkbar, dass das Wirkelement aufgrund seiner äußeren Geometrie durch die Rotationsbewegung einen Unterdruck - vergleichbar einer Turbinenschaufel - erzeugt. Linsentrümmer könnten somit effektiver in den Absaugkanal der Hohlnadel gesogen werden.

Das Wirkelement kann beispielsweise als Kreisring ausgebildet sein. Der Kreisring kann über eine Wulst oder eine lineare Vertiefung in der Mantelfläche des Absaugkanals befestigt sein. Hierbei ist es denkbar, mehrere Ringe hintereinander vorzusehen. In vorteilhafter Weise ist mindestens ein Steg vorgesehen, der sich entlang einer Sehne des Kreisrings erstreckt. Dabei kann der Steg insbesondere entlang des Durchmessers des Kreisrings verlaufen.

In weiter vorteilhafter Weise sind mindestens zwei Stege vorgesehen, die sich von einem Mittelelement radial zum Kreisring hin erstrecken.

Das Wirkelement kann in weiter vorteilhafter Weise als mindestens ein sich senkrecht zur Mantelfläche des Absaugkanals erstreckender Steg ausgebildet sein. Des Weiteren ist denkbar, dass das Wirkelement aus mehreren Stegen besteht, die sich von einem Mittelelement radial zur Mantelfläche des Absaugkanals hin erstrecken.

Das Mittelelement weist in besonders vorteilhafter Weise die Form einer Kugel oder eines Kugelsegments oder einer Pyramide auf. Hierbei sei angemerkt, dass das Mittelelement auch eine unregelmäßige, d.h. unsymmetrische geometrische Form aufweisen kann.

Grundsätzlich ist es denkbar, dass die Oberfläche des Wirkelements, d.h. des Stegs bzw. der Stege und/oder des Mittelelements und/oder des Kreisrings, glatt ausgebildet ist. Auch ist es denkbar, dass die Oberfläche Strukturen, insbesondere Pyramiden, Kegel oder Zylinder aufweist. Schließlich kann die Oberfläche auch gewellt, gezackt oder gezahnt ausgebildet sein. Durch die verschiedenen Möglichkeiten die Oberfläche des Wirkelements auszugestalten, kann auf die mechanische Zertrümmerung der Linsen und die Emittierung der Ultraschallwellen Einfluss genommen werden.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung fünf bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht das freie Ende einer Hohlnadel, wie sie aus dem Stand der Technik bekannt ist,
- Fig. 2: in einer schematischen Ansicht, vergrößert, den Arbeitsbereich eines ersten Ausführungsbeispiels einer erfindungsgemäßen Hohlnadel,
- Fig. 3: in einer schematischen Ansicht, vergrößert, den Arbeitsbereich eines zweiten Ausführungsbeispiels einer erfindungsgemäßen Hohlnadel,
- Fig. 4: in einer schematischen Ansicht, vergrößert, den Arbeitsbereich eines dritten Ausführungsbeispiels einer erfindungsgemäßen Hohlnadel,
- Fig. 5: in einer schematischen Ansicht, vergrößert, den Arbeitsbereich eines vierten Ausführungsbeispiels einer erfindungsgemäßen Hohlnadel und
- Fig. 6: in einer schematischen Ansicht, vergrößert, den Arbeitsbereich eines fünften Ausführungsbeispiels einer erfindungsgemäßen Hohlnadel.

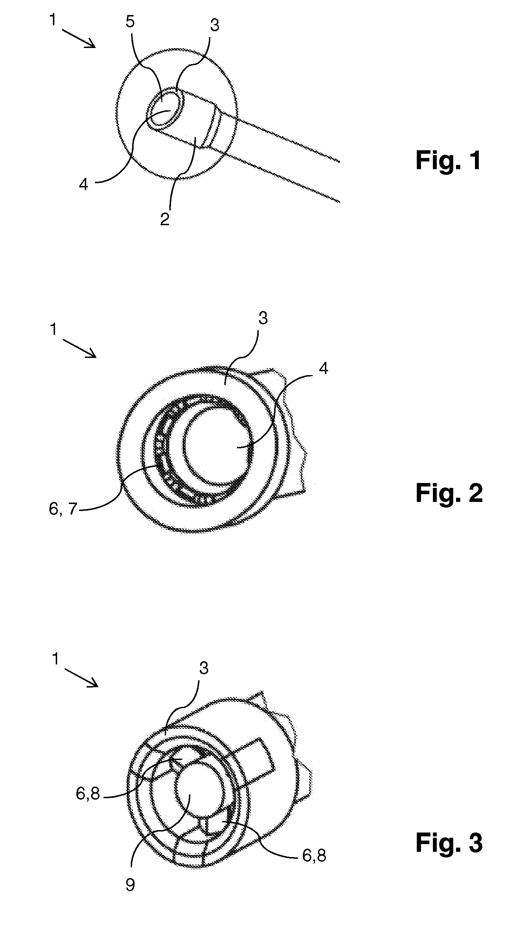

Fig. 1 zeigt das freie Ende 1 einer Hohlnadel für ein augenchirurgisches Instrument zur In-vivo-Zertrümmerung organischer Linsen mittels Ultraschall, wie sie aus dem Stand der Technik bekannt ist. Die Hohlnadel umfasst ein am freien Ende 1 ausgebildeten Arbeitsbereich 2. Der Arbeitsbereich 2 dient zum Emittieren von Ultraschallwellen, wobei sich durch die Hohlnadel hindurch ein im Arbeitsbereich 2 offener Absaugkanal 4 zum Absaugen von Linsentrümmern erstreckt. Die Öffnung 5 des Absaugkanals 4 ist durch die Wirkfläche 3 gebildet bzw. begrenzt.

Fig. 2 zeigt ein erstes Ausführungsbeispiel einer erfindungsgemäßen Hohlnadel. In der Mantelfläche des Absaugkanals 4 ist das Wirkelement 6 in Form eines Kreisrings 7 eingebracht. Im hier dargestellten Ausführungsbeispiel weist die Mantelfläche des Absaugkanals 4 eine lineare Vertiefung in Form einer Nut auf. In diese Vertiefung ist der Kreisring 7 derart eingebracht, dass er gegenüber der Hohlnadel beweglich ist. Durch die Bewegung des freien Endes 1 der Hohlnadel wird der Kreisring 7 in Rotation versetzt. Die Oberfläche des Kreisrings 7 ist mit konvexen pyramidenförmigen Geometrien versehen. Die Rotation des Kreisrings 7 führt zu einer verbesserten mechanischen Zertrümmerung der organischen Linse. Aufgrund der Oberflächenstruktur des Kreisrings 7 wird außerdem eine größere Fläche zum emittieren von Ultraschallwellen geschaffen, was die Effizienz der Zerkleinerung organischer Linsen ebenfalls erhöht.

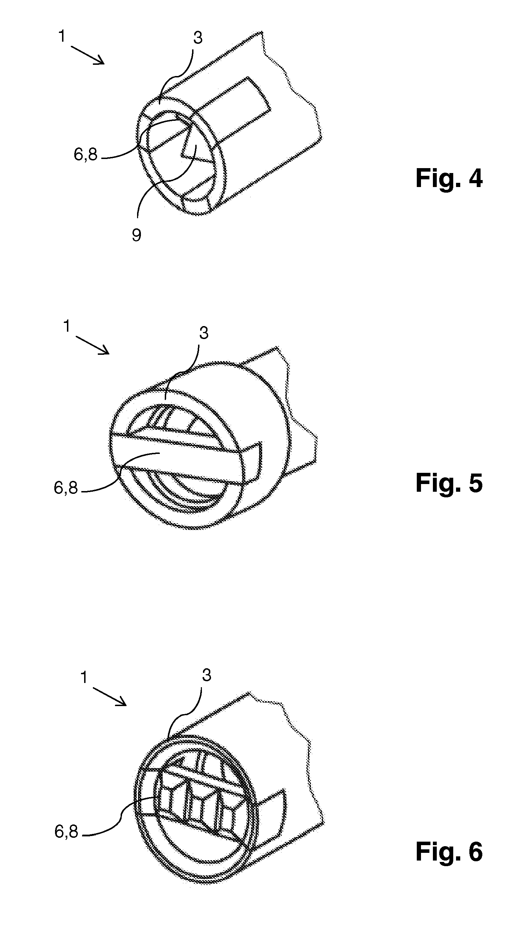

Fig. 3 zeigt ein weiteres Ausführungsbeispiel einer erfindungsgemäßen Hohlnadel. Das Wirkelement 6 ist durch drei Stege 8, die radial vom Mittelelement 9 zur Mantelfläche des Absaugkanals 4 verlaufen, gebildet. Das Mittelelement hat die Form eines Kugelsegments. Fig. 4 entspricht der Ausführung aus Fig. 3, wobei hier das Mittelelement 9 in Form einer Pyramide gebildet ist.

In den Fig. 5 und 6 ist das Wirkelement 6 aus einem Steg 8 gebildet. Der Steg 8 läuft entlang des Durchmessers des Absaugkanals 4. In Fig. 5 ist die Oberfläche des Stegs 8 glatt ausgebildet. Fig. 6 zeigt eine prismenartige Struktur auf der Oberfläche des Stegs 8. Durch die Struktur wird die ultraschallemittierende Fläche vergrößert.

In Bezug auf Merkmale, die sich den Figuren nicht entnehmen lassen, sei zur Vermeidung von Wiederholungen auf den allgemeinen Teil der Beschreibung verwiesen.

Schließlich sei ausdrücklich darauf hingewiesen, dass die voranstehend beschriebenen Ausführungsbeispiele der erfindungsgemäßen Hohlnadel lediglich zur Erörterung der beanspruchten Lehre dienen, diese jedoch nicht auf die Ausführungsbeispiele einschränken.

### Bezugszeichenliste

- 1: freies Ende
- 2: Arbeitsbereich
- 3: Wirkfläche
- 4: Absaugkanal
- 5: Öffnung
- 6: Wirkelement
- 7: Kreisring
- 8: Steg
- 9: Mittelelement

## Patentansprüche

1. Hohlnadel für ein augenchirurgisches Instrument zur In-vivo-Zertrümmerung organischer Linsen mittels Ultraschall, mit einem Anschlussbereich zum Ankoppeln an das Instrument und einem am freien Ende (1) der Hohlnadel ausgebildeten Arbeitsbereich (2) mit einer Wirkfläche (3) zum Abstrahlen von Ultraschallwellen, wobei sich durch die Hohlnadel hindurch ein im Arbeitsbereich (2) offener Absaugkanal (4) zum Absaugen von Linsentrümmern erstreckt, dessen Öffnung (5) durch die Wirkfläche (3) gebildet ist, wobei im Arbeitsbereich (2) mindestens ein Wirkelement (6) vorgesehen ist, das sich zumindest geringfügig in den Absaugkanal (4) hinein erstreckt,
**dadurch gekennzeichnet, dass** das Wirkelement (6) gegenüber der Hohlnadel beweglich ist.

2. Hohlnadel Anspruch 1, **dadurch gekennzeichnet, dass** das Wirkelement (6) als Kreisring (7) ausgebildet ist.

3. Hohlnadel nach Anspruch 2, **dadurch gekennzeichnet, dass** mindestens ein Steg (8) vorgesehen ist, der sich entlang einer Sehne, insbesondere des Durchmessers, des Kreisrings (7) erstreckt.

4. Hohlnadel nach Anspruch 2, **dadurch gekennzeichnet, dass** mindestens zwei Stege (8) sich von einem Mittelelement (9) radial zum Kreisring (7) hin erstrecken.

5. Hohlnadel nach Anspruch 1, **dadurch gekennzeichnet, dass** das Wirkelement (6) als mindestens ein sich senkrecht zur Mantelfläche des Absaugkanals (4) erstreckender Steg (8) ausgebildet ist.

6. Hohlnadel nach Anspruch 1, **dadurch gekennzeichnet, dass** das Wirkelement (6) aus mehreren Stegen (8) ausgebildet ist, die sich von einem Mittelelement (9) radial zur Mantelfläche des Absaugkanals (4) erstrecken.

7. Hohlnadel nach Anspruch 4 oder 5, **dadurch gekennzeichnet, dass** das Mittelelement (9) in Form einer Kugel oder eines Kugelsegments oder einer Pyramide ausgebildet ist.

8. Hohlnadel nach einem der Ansprüche 2 bis 7, **dadurch gekennzeichnet, dass** die Oberfläche des Stegs (8) bzw. der Stege (8) und/oder des Mittelelements (9) und/oder des Kreisrings (7) glatt ausgebildet ist.

9. Hohlnadel nach einem der Ansprüche 2 bis 7, **dadurch gekennzeichnet, dass** die Oberfläche des Stegs (8) bzw. der Stege (8) und/oder des Mittelelements (9) und/oder des Kreisrings (7) Strukturen, insbesondere Pyramiden, Kegel oder Zylinder, aufweist.

10. Hohlnadel nach einem der Ansprüche 2 bis 7, **dadurch gekennzeichnet, dass** die Oberfläche des Stegs (8) bzw. der Stege (8) und/oder des Mittelelements (9) und/oder des Kreisrings (7) gewellt, gezackt oder gezahnt ausgebildet ist.

## Claims

1. Hollow needle for an ophthalmic surgical instrument for in-vivo fragmentation of organic lenses using ultrasound, having a connection region for coupling to the instrument and an operating region (2) which is formed at the free end (1) of the hollow needle and which has an active face (3) for radiating ultrasound waves, wherein there extends through the hollow needle a suction channel (4) which is open in the operating region (2) for suction of lens debris and whose opening (5) is formed by the active face (3), wherein there is formed in the operating region (2) at least one active element (6) which extends at least slightly into the suction channel (4),
**characterised in that** the active element (6) can be moved with respect to the hollow needle.

2. Hollow needle according to claim 1, **characterised in that** the active element (6) is constructed as a circular ring (7).

3. Hollow needle according to claim 2, **characterised in that** there is provided at least one web (8) which extends along a chord, in particular of the diameter, of the circular ring (7).

4. Hollow needle according to claim 2, **characterised in that** at least two webs (8) extend from a central element (9) radially towards the circular ring (7).

5. Hollow needle according to claim 1, **characterised in that** the active element (6) is constructed as at least one web (8) which extends perpendicularly relative to the covering face of the suction channel (4).

6. Hollow needle according to claim 1, **characterised in that** the active element (6) is formed by a plurality of webs (8) which extend from a central element (9) radially with respect to the covering face of the suction channel (4).

7. Hollow needle according to claim 4 or 5, **characterised in that** the central element (9) is constructed in the form of a ball or a spherical segment or a pyramid.

8. Hollow needle according to any one of claims 2 to 7, **characterised in that** the surface of the web (8) or the webs (8) and/or the central element (9) and/or the circular ring (7) is/are constructed in a smooth manner.

9. Hollow needle according to any one of claims 2 to 7, **characterised in that** the surface of the web (8) or the webs (8) and/or the central element (9) and/or the circular ring (7) has structures, in particular pyramids, cones or cylinders.

10. Hollow needle according to any one of claims 2 to 7, **characterised in that** the surface of the web (8) or the webs (8) and/or the central element (9) and/or the circular ring (7) is constructed in an undulating, serrated or toothed manner.

## Revendications

1. Aiguille creuse pour un instrument de chirurgie oculaire destiné à la fragmentation in vivo du cristallin organique par ultrasons, comportant une zone de raccordement pour le raccordement à l'instrument et une zone de travail (2), réalisée au niveau de l'extrémité libre (1) de l'aiguille creuse et munie d'une surface active (3) pour émettre les ondes ultrasonores, un conduit d'aspiration (4) ouvert, destiné à aspirer les fragments de cristallin, s'étendant à travers l'aiguille creuse dans la zone de travail (2), l'ouverture (5) dudit conduit d'aspiration étant formée par la surface active (3), au moins un élément actif (6) étant prévu dans la zone de travail (2) et s'engageant au moins légèrement dans le conduit d'aspiration (4),
**caractérisée en ce que** l'élément actif (6) est mobile par rapport à l'aiguille creuse.

2. Aiguille creuse selon la revendication 1, **caractérisée en ce que** l'élément actif (6) est réalisé sous la forme d'une bague circulaire (7).

3. Aiguille creuse selon la revendication 2, **caractérisée en ce qu'**il est prévu au moins une barrette (8) qui s'étend le long d'une corde, en particulier le diamètre, de la bague circulaire (7).

4. Aiguille creuse selon la revendication 2, **caractérisée en ce qu'**au moins deux barrettes (8) s'étendent depuis un élément central (9) radialement vers la bague circulaire (7).

5. Aiguille creuse selon la revendication 1, **caractérisée en ce que** l'élément actif (6) est réalisé sous la forme d'au moins une barrette (8) qui s'étend perpendiculairement à la paroi latérale du conduit d'aspiration (4).

6. Aiguille creuse selon la revendication 1, **caractérisée en ce que** l'élément actif (6) est constitué de plusieurs barrettes (8) qui s'étendent depuis un élément central (9) radialement vers la paroi latérale du conduit d'aspiration (4).

7. Aiguille creuse selon la revendication 4 ou 5, **caractérisée en ce que** l'élément central (9) est réalisé sous la forme d'une sphère ou d'un segment de sphère ou d'une pyramide.

8. Aiguille creuse selon l'une quelconque des revendications 2 à 7, **caractérisée en ce que** la surface de la barrette (8) ou des barrettes (8) et/ou de l'élément central (9) et/ou de la bague circulaire (7) est lisse.

9. Aiguille creuse selon l'une quelconque des revendications 2 à 7, **caractérisée en ce que** la surface de la barrette (8) ou des barrettes (8) et/ou de l'élément central (9) et/ou de la bague circulaire (7) comporte des structures, en particulier des pyramides, des cônes ou des cylindres.

10. Aiguille creuse selon l'une quelconque des revendications 2 à 7, **caractérisée en ce que** la surface de la barrette (8) ou des barrettes (8) et/ou de l'élément central (9) et/ou de la bague circulaire (7) est ondulée, dentelée ou dentée.
